# EUROPEAN PATENT APPLICATION

(11) **EP 2 886 130 A1**
(43) Date of publication of application: **24.06.2015**
(21) Application number: 14004387.8
(22) Date of filing: 23.12.2014
(51) Int. Cl.: A61K 47/02, A61K 47/12, A61K 31/352, A61K 31/382, A61K 31/498, A61K 31/5377, A61K 31/5383, A61K 31/5575, A61P 27/02

(54) **Ophthalmic pharmaceutical composition and process for the preparation thereof**

(30) Priority: 23.12.2013 GR 20130100709
(71) Applicant: RAFARM S.A., 154 51 Neo Psihiko, Athens (GR)
(72) Inventor: Rassia, Ioanna, 190 02 Peania Attikis (GR); Chalkias, George, 190 02 Peania Attikis (GR)

(57) **Abstract**

The present invention relates to an ophthalmic pharmaceutical composition for topical administration comprising a therapeutically effective quantity of an active ingredient and/or combination with another active ingredient more particular said active ingredient is selected from a prostaglandin or a pharmaceutically acceptable salt or derivative thereof, a fluoroquinolone such as Levofloxacin and salts or derivatives thereof, a carbonic anhydrase inhibitor, such as Dorzolamide or salts thereof, a beta-adrenergic receptor antagonist, such as Timolol or salts thereof, a mast cell stabilizer such as cromoglicic acid or salts thereof, an α₂-adrenoceptor antagonist such as brimonidine or salts thereof and/or combination of said active ingredients for the treatment of a condition affecting the exterior ocular surface, and a process for the preparation thereof, which exhibits enhanced corneal epithelial permeability.

## Description

### TECHNICAL FIELD OF THE INVENTION

The present invention relates to an ophthalmic pharmaceutical formulation for topical administration comprising a therapeutically effective quantity of an active ingredient, in particular a prostaglandin or a pharmaceutically acceptable salt or derivative thereof, a fluoroquinolone such as Levofloxacin and salts or derivatives thereof, a carbonic anhydrase inhibitor, such as Dorzolamide or salts thereof, a beta-adrenergic receptor antagonist, such as Timolol or salts thereof, a mast cell stabilizer such as cromoglicic acid or salts thereof, an α₂-adrenoceptor antagonist such as brimonidine or salts thereof and/or combination of said active ingredients, for the treatment of conditions affecting the exterior eye surface and a process for the preparation thereof.

### BACKGROUND OF THE INVENTION

Ophthalmic solutions are mainly sterile clear aqueous solutions free of solid particles, suitably packaged for direct instillation in the eye. The essential feature of the ophthalmic solutions is that all ingredients (active ingredient and excipients) are fully dissolved in the aqueous medium and does not comprise any form of visible insoluble particles.

One of the fibrous tissues surrounding the eye is the cornea. It is well known that the absorption of most drugs occurs primarily through the cornea, and therefore the permeability of the corneal tissue is considered the greatest biological barrier for ophthalmic preparations. Generally, the cornea acts as a storehouse of the active ingredient of ophthalmic compositions for topical application.
The basic structure of the cornea consists of three main layers, namely the epithelium, the stroma and the endothelium. Each layer has different structural characteristics. The epithelium comprises about 90% of the cellular material of the corneal tissue and for this reason it is the most lipophilic part (composed of a large proportion of cell membranes). The links between the cells of the epithelium is highly cohesive thereby making the permeability therethrough difficult. The epithelium layer accounts for 9 % of the thickness of the corneal tissue, however it is the greatest obstacle in the permeability rate/absorption of the active ingredients.
The stroma layer occupies 90 % of the thickness of the cornea and is the basic storage for hydrophilic active substances. Finally, the endothelium layer due to the very small thickness (only 1% of the cornea) is not considered as a serious barrier for the permeation of active substances.
How fast and how much the drug will penetrate the cornea and reach inside the bulb depends on many factors related to the physicochemical characteristics of the active ingredient and the solution comprising said active ingredient.

Various methods are already known for the industrial preparation of oral dosage forms comprising prostaglandin or a pharmaceutical acceptable salt or derivative thereof, as an active ingredient due to its useful therapeutical properties. However, the prior art has encountered substantial difficulties in the production of stable ophthalmic compositions of prostaglandin. Prostaglandins are susceptible to adsorption/absorption by plastic packaging components, and this adsorption/absorption decreases the potency of said compositions upon storage.

EP 1 753 434 discloses a stable composition of bimatoprost wherein in order to enhance the permeability of Bimatoprost ophthalmic solution, benzalkonium chloride (BAC) is added as a preservative and provides comparison in vivo studies between BAC and BAC-free Bimatoprost formulations on ocular absorption of the latter.
N. Keller et al, "Increased Corneal Permeability Induced by the Dual Effects of Transient Tear Film Acidification and Exposure to Benzalkonium Chloride" Exp. Eye.Res.(1980) 30, 203-210, it is disclosed that BAC combined with acid buffering enhance in a considerable amount corneal permeability of an ophthalmic drug.

Although each of the above documents represents an attempt to overcome the stability and permeability problems associated with pharmaceuticals compositions comprising prostaglandins, there still exists a need for improving the stability and permeability of such pharmaceutical compositions.

### SUMMARY OF THE INVENTION

It is, therefore, an object of the present invention to provide an ophthalmic pharmaceutical composition for topical administration comprising an active ingredient, such as prostaglandin or a pharmaceutically acceptable salt or derivative thereof, a fluoroquinolone such as Levofloxacin and salts or derivatives thereof, a carbonic anhydrase inhibitor, such as Dorzolamide or salts thereof, a beta-adrenergic receptor antagonist, such as Timolol or salts thereof, a mast cell stabilizer such as cromoglicic acid or salts thereof, an α₂-adrenoceptor antagonist such as brimonidine or salts thereof and/or combination of said active ingredients, for the treatment of conditions affecting the exterior eye surface, which overcomes the deficiencies of the prior art.

Moreover, it is another object of the present invention to employ a suitable manufacturing process enhancing the permeability of the active ingredient used in an ophthalmic formulation, and in particular comprising prostaglandin or a pharmaceutically acceptable salt or derivative thereof a fluoroquinolone such as Levofloxacin and salts or derivatives thereof, a carbonic anhydrase inhibitor, such as Dorzolamide or salts thereof, a beta-adrenergic receptor antagonist, such as Timolol or salts thereof, a mast cell stabilizer such as cromoglicic acid or salts thereof, an α₂-adrenoceptor antagonist such as brimonidine or salts thereof and/or combination of said active ingredients, for the treatment of conditions affecting the exterior eye surface.

In accordance with the above objects of the present invention, an ophthalmic pharmaceutical composition for topical administration is provided comprising a therapeutically effective quantity of an active ingredient and/or combination with another active ingredient for the treatment of conditions affecting the exterior eye surface, wherein it comprises a prostaglandin or a pharmaceutically acceptable salt or derivative thereof, a fluoroquinolone such as Levofloxacin and salts or derivatives thereof, a carbonic anhydrase inhibitor, such as Dorzolamide or salts thereof, a beta-adrenergic receptor antagonist, such as Timolol or salts thereof, a mast cell stabilizer such as cromoglicic acid or salts thereof, an α₂-adrenoceptor antagonist such as brimonidine or salts thereof as active ingredient and/or combination of said active ingredients, and effective amount of agents for the optimization of the drop size, the pH value, the osmolality and viscosity in order to improve the stability and permeability properties of the composition, and without the use of any antimicrobial preservative compound such as benzalkonium chloride.

According to another embodiment of the present invention, a process for the preparation of an ophthalmic pharmaceutical composition for topical administration is provided comprising a therapeutically effective quantity of an active ingredient and/or combination with another active ingredient for the treatment of a condition affecting the exterior ocular surface, wherein it comprises prostaglandin or a pharmaceutically acceptable salt or derivative thereof, a fluoroquinolone such as Levofloxacin and salts or derivatives thereof, a carbonic anhydrase inhibitor, such as Dorzolamide or salts thereof, a beta-adrenergic receptor antagonist, such as Timolol or salts thereof, a mast cell stabilizer such as cromoglicic acid or salts thereof, an α₂-adrenoceptor antagonist such as brimonidine or salts thereof as active ingredient and/or combination of said active ingredients, and effective amount of agents for the optimization of the drop size, the pH value, the osmolality and viscosity in order to improve the stability and permeability properties of the composition, and without the use of any antimicrobial preservative compound such as benzalkonium chloride, said composition is being sterilized by aseptic filtration through membrane filter.
Further preferred embodiments of the present invention are defined in dependent claims 2 to 4.

Other objects and advantages of the present invention will become apparent to those skilled in the art in view of the following detailed description.

### DETAILED DESCRIPTION OF THE INVENTION

During the development of ophthalmic solutions certain parameters must be taken into account in order to prepare a pharmaceutical composition possessing certain levels of stability, purity, bioavailability and being suitable for safe and efficacious administration to patients. Said parameters comprise the concentration levels of the active ingredient in the solution, the ionization constant of the active ingredient, the distribution coefficient of the active ingredient, the influence of the pH value, the osmotic pressure of the solution, the type and capacity of the buffer system (buffer capacity), the viscosity of the solution, the surface tension, the preservative, and the sterilization technology.

The exact amount of active ingredient to be absorbed by the eye and potentially to be therapeutic effective depends on the amount of active ingredient applied to the surface of the eye during the administration of the formulation (i.e. the concentration of the active substance in ophthalmic solution) and by the permeability of the active ingredient wherein the permeability of a substance is defined as the percentage of the administered amount (that is instilled in the eye) which ultimately absorbed (i.e. that has penetrated the corneal tissue). In any case, the concentration of the active substance in the ophthalmic solution should ensure both the uniformity and stability of the formulation and the desired therapeutic effect through administration of a particular solution volume (e.g. number of drops) into the eye. Furthermore, an active ingredient, when dissolved in a solution, can exist in two forms, namely in ionised and non - ionised form. The ionised and non - ionised form of an active ingredeint in a solution are in equilibrium with each other. The ionization constant of a substance, pKa, is defined as the negative logarithm of the constant Ka and denotes the equilibrium ionization of the molecule. The ionization constant number is unique and identifies each compound. The degree of ionization of a substance significantly affects the solubility of said substance in solution. It is widely known that the ionized form of an active ingredient exhibits better solubility than the non - ionized form. Therefore, a greater degree of ionization leads to better solubility. Consequently, the ionization constant pKa affects the solubility of the substance in a solution.

Moreover, the distribution coefficient K_{ow} of the active ingredient, namely the octanol - water, K_{ow}, is the ratio of the solubility of a chemical in 1- octanol and water at equilibrium. The K_{ow} value of an active ingredient indicates in a quantitative manner the hydrophilicity and hydrophobicity of said active ingredient. The lipophilic and hydrophilic character of a substance affects its permeability thereof through the tear fluid and the various layers of the cornea. Generally, the hydrophilic character of a substance (i.e. a small value of K_{ow}) enhances the permeation through the lacrimal fluid and through the stroma of the cornea and the hydrophobic character of a substance (i.e. high value of K_{ow}) favors permeation through the epithelium.

Furthermore, it is widely known that the pH value of the solution depends from the solubility of the active substance, the degree of ionization of the active substance and the stability of the active substance.
Thus, in an ophthalmic formulation the pH value should be adjusted to a value so as to ensure the solubility of the selected level of concetration in the solution and the stability of the active ingredeint over time. In addition, the pH value is also an important determinant factor of the permeability of the active ingredient through the corneal tissue.
The amount of active ingredient in ionized form depends from the pH value of the solution, wherein said ionized form of the active ingredient has slower and lower permeability through the cornea than the non - ionized form of the active substance.

In general, it has been shown that many active substances are more effective (i.e. exhibit greater permeability) at pH values at which the active ingredient has a higher degree of non-ionized form. In most cases, however, there is a large discrepancy between the pH values favoring the solubility and/or stability than those that favor the permeability of the active ingredient.
For this reason, during the development of an ophthalmic solution it should be combined in order to optimize satisfactorily all the desired characteristics.

It has been found that isotonic solutions with a pH value not within the normal pH range, cause disorders such as dryness, irritation, tear production, photophobia and discomfort, upon ocular administration.
Generally, solutions with pH value from about 7 to about 8 have very good compatibility with the eye, solutions with pH value higher than about > 9 cause discomfort while solutions with pH value from about 4.5 to about 6 is expected to cause irritation or discomfort. Thus, we have found according to the present invention that the pH of the ophthalmic solutions of the present invention should be adjusted to a range from about 4 to 8 in order to not vary significantly from the normal pH value (i.e. pH = 7.4) of the lachrymal fluid which would cause severe irritation of the eye during application.
If the pH value of an ophthalmic formulation is outside the acceptable range, even a drop would be sufficient to significantly alter the pH of lacrimal fluid during application and thus lead to eye irritation and additional lacrimal fluid secretion due to defense mechanism of the eye which would reduce the inductive effect of the drug.

The osmolality of a solution is also an important factor that needs to be considered. The osmotic pressure of a solution is a cumulative property, which depends on the number of particles of the dissolved components in the solution. Biological fluids including blood and lacrimal fluid have the same osmotic pressure as a sodium chloride concentration of 0.9% (about 300mOsm/L, 280-310 mOsm/L). All solutions having an osmotic pressure equal to a solution of 0.9% sodium chloride are characterized as isotonic. The solutions with an osmotic pressure higher or below than the above value, are characterized hypertonic and hypotonic, respectively.
When applying hypotonic or hypertonic solutions to the eye, the corneal tissue cells coming into contact with the respective ophthalmic solution perceive a difference in osmotic pressure. Accordingly, ocular administration of hypertonic and hypotonic solutions entail osmotic imbalance and mobility of water molecules through the cell membrane, causing irritation, discomfort, tear production and photophobia.

Therefore, an ophthalmic solution should ideally be isotonic with the lacrimal fluid. It is well known that the eye can tolerate (without causing great discomfort) solutions with osmotic pressure corresponding to the sodium chloride concentrations from 0.6 % to 2%.
Hypotonic or hypertonic solutions, upon contact with the eye may change the surface of cells of the corneal tissue and expand it to affect the permeability of the tissue from the active ingredient. Since the volume of the ophthalmic formulation that is used is small, the dilution with the lachrymal fluid can reverse the potential hypertonicity of the solution. In fact, the osmolality of the ophthalmic products is adjusted to the desired value (which should be close to the value of the tear fluid), by adding suitable agents. The most widely used osmotic agents are sodium chloride and dextrose or maltose. Since all components dissolved in a solution contribute to the osmotic pressure of the solution, according to the present invention during the osmotic pressure adjustment of the final solution by adding a suitable osmotic agent, the contribution of all other components of the solution should be considered.

Furthermore, the use of a buffer in an ophthalmic solution is very important as through the use of buffer it is ensured the stability and solubility of the active ingredient throughout life (shelf life) of the product.
It is generally known, that the solubility and stability of a substance vary significantly as a function of pH. So when developing an ophthalmic solution conducted a series of in vitro studies determined the pH at which the active substance exhibits good solubility (for the desired concentration levels) and also remains constant over time. Any change in pH value would cause precipitation and/or degradation of the active.

The main buffer systems, which are used in pharmaceutical systems, are salts of phosphoric acid and carbonic acid as well as salts of citric acid. Generally, during the development of an ophthalmic solution, the choice of a buffer is determined by the compatibility of the active ingredient of the solution. In other words, it is selected a buffer system in which the active ingredient has a good stability and does not deteriorate over time.

The capacity of a buffer system depends on the type of the regulatory system, and the concentration of the components of the buffer solution. It is well known that that phosphate buffers, citrate and acetate salts of the same concentration show different capacity. It is clear that the drug solution, when instilled in the eye, is mixed with the lacrimal fluid and thus it alters its characteristics (e.g. the quantitative and qualitative composition). Accordingly, the solution of the active ingredient that comes in direct contact with the eye has a different pH value of said solution in the container.
Generally, the buffer capacity of an ophthalmic solution should not overcome the capacity of the lacrimal fluid (which has the capacity to restore and maintain the normal pH value at 7.4) in order to avoid phenomena of irritation and discomfort due to prolonged change of pH in the eye region.
It has also been found according to the present invention that when the buffer capacity is high, this means that the drug solution shows less absorption and less permeability.

In addition, another factor that needs to be considered is the viscosity. Viscosity is a physical property of the liquid which is defined as the measure of resistance of a fluid to flow. Solutions with high viscosity have little flow and are characterized as viscous while solutions with low viscosity show strong flow and are characterized as free-flowing.
Topical ophthalmic preparations are inhibited by certain mechanisms deployed in the area of drug instillation and thus, only a small percentage of the administered dose passes through the cornea and penetrates the interior of the eye, leading to low permeability of the active ingredient. Important role in the absorption of the active ingredient of topical ophthalmic preparations plays the contact time and prolongation of the contact time of ophthalmic solution in the eye.

Thus, in order to increase the absorption of the active ingredient, the contact time of the solution with the corneal tissue should be prolonged.
Consequently, increase of the viscosity of an ophthalmic solution, by adding to the formulation of suitable viscosity enhancing agents, cause a reduced flow of liquid in the application to the eye, prolongation of the contact time with the cornea tissue and eventually increase the absorption of the active substance.

As viscosity enhancing agents are used primarily water soluble polymers such as hydroxyl ethyl cellulose, hydroxylpropyl methylcellulose, polyvinyl alcohol and carboxy methylcellulose. However, the increase of the viscosity of an ophthalmic solution above the normal value, namely above the viscosity value of the lacrimal fluid, may function negatively. Thus, although the increase in viscosity prolongs the contact time and increases the absorption, it can both delay the diffusion of the active substance in solution and can cause problems when mixed with the tear fluid. Additionally, due to the polymer adsorption on the surface of the eye it can prevent the penetration of the drug.
In addition, the application of ophthalmic solutions with high viscosity may cause tearing, blurring and blinking due to the effort of the eye to regain normal viscosity. There are several conflicting investigations known regarding the optimum viscosity value in relation to the permeability of the ophthalmic solutions.
According to the present invention, the viscosity of an ophthalmic solution should range from about 70 to about 130 cP in order to have safe use and optimal therapeutic effect.

The surface tension is another parameter that needs to be considered. Surface tension is called the force that must be applied to a surface in order to overcome the internal attractive forces. Surface tension is a property of the surface of the liquid, which allows them to resist external forces. It is obvious that a liquid with high surface tension will show less tendency to wet the surface of a liquid than with low surface tension.
Therefore, each component which reduces the surface tension, it also increases the wettability and liquid penetration. The permeability of many ophthalmic drugs through the corneal tissue is limited and thus, the therapeutic effect is less than desirable.
However, it has been proven that the use of suitable surfactants (e.g. wetting agents and surfactants) may lead to a substantial increase of the permeability of the active ingredient which is attributed to the reduction of surface tension of the solution.
Reduction of the surface tension in an ophthalmic solution causes an increase of the surface area wetted by the solution during ophthalmic use, thereby ensuring better contact of the solution with the tear fluid on the eye surface and thus increases the absorption of the active substance. The surface tension of the solution is adjusted by adding to the formulation of a suitable surfactant such as surfactants and wetting agents. The main characteristic of these excipients is that they are amphiphilic in nature in that they comprise a hydrophilic and a hydrophobic portion.

Still another parameter that should be considered is the use of a preservative. Preservatives are substances or mixtures of substances added to the ophthalmic solutions with the aim of preventing the growth or destroy microorganisms contaminating the formulation during use. The preservatives are used in ophthalmic products packaged in multi-dose containers, except if the drug itself possesses antimicrobial activity. Also, it is not necessary to use preservatives in monodose dosage forms. Generally, the use of preservative should be restricted to the minimum possible as preservatives can lead to irritation and/or destruction of sensitive eye tissues.

The most commonly used preservative is benzalkonium chloride, which is used in 70 % of the commercially available ophthalmic formulations. Although it has been challenged due to hypersensitivity indications and toxicity, it is stable in solution, stable during sterilization and at a concentration of 0.01 % it is effective throughout the pH range used in ophthalmic solutions. Other preservatives used in ophthalmic solutions are parabens, chlorobutanol, chlorhexidine, phenylethanol and the organomercurial compound thimerosal.

Moreover, the method of sterilization used should be optimized. Sterilization is called the set of methods and tools that are intended to remove or destroy all forms of microbial life and their seeds present on a surface, equipment or pharmaceutical.

The main methods of sterilization that may be used are by heat, by chemical sterilization with ethylene oxide, by irradiation and by filtration.

Generally, the choice of the method for the sterilization of ophthalmic solutions should be based on specific criteria so as to ensure the efficiency of the process (production of a sterile product) and the stability of the active substance.
The pharmaceutical compositions of the present invention are considered stable if the concentration of active ingredient can be maintained at the level specified on the label for the maximum anticipated shelf-life under given environmental conditions. A pharmaceutical composition is considered unstable when the potency of the active ingredient is lost.

The potency of a drug product may decline over time during storage due to various reasons, such as degradation of the active ingredient, reaction of the active ingredient with excipients or container closure components, leaching of the active ingredient through the container wall, and absorption/adsorption of the active ingredient into/to the container wall. Similarly, the purity of a medicinal preparation may also change during storage due to leaching of chemicals into the drug preparation from the container materials, from the labels on the containers, or from the environment where the packaged medicinal product is stored. Thus, the containers used for packaging medicinal preparations may significantly affect the stability and purity of the preparations contained therein.

It has been surprisingly found that the object of the present invention is achieved by employing one of the following active ingredients and/or combination thereof: a prostaglandin or a pharmaceutically acceptable salt or derivative thereof, a fluoroquinolone such as Levofloxacin and salts or derivatives thereof, a carbonic anhydrase inhibitor, such as Dorzolamide or salts thereof, a beta-adrenergic receptor antagonist, such as Timolol or salts thereof, a mast cell stabilizer such as cromoglicic acid or salts thereof, an α₂-adrenoceptor antagonist such as brimonidine or salts thereof and/or combination of said active ingredients, and by optimization of the drop size, the pH value, the osmolality and viscosity and by avoiding the use of any antimicrobial preservative compound in order to improve the stability and permeability properties of the composition.

Prostaglandin ophthalmic formulations for topical administration are known to undergo loss in potency of the active ingredient due to adsorption from the materials used throughout the whole manufacturing process of said formulation. According to the present invention, in order to facilitate the process for the preparation of the prostaglandin composition, a compatible filter membrane may be incorporated, which eliminates the inline retention of the active ingredient, on the membrane filters used during the aseptic filtration procedure.

Plastic containers have some drawbacks since the active agent, depending on the choice of plastic material employed for the container, can be absorbed and/or adsorbed by the container material.
In general, ophthalmic medicaments are marketed in mono-dose or multi-dose dispensers, the latter being of preference due to their ease of use and cost effectiveness.
In such multi-dose dispensers, in order to prevent degradation of the product and avoid the spread of potentially harmful bacteria, the content has to be kept sterile, both during storage and while in use by the patient or consumer. To meet said need the inclusion of preservatives like BAC is common practice.

However, the high incidence of local side effects attributed to preservatives is of significance for the patient and the relevant Health Authorities as discussed in the recent years. Despite the fact that already published preclinical and clinical studies are not always consistent, it seems to be clear that short-term use of preparations containing preservatives at low concentrations is well tolerated, but preservatives can cause serious inflammatory effects such as chemical irritation, hyper-reactivity and true allergies, in long-term use.

Systems allowing multi-dose use of preservative-free formulations have the potential to solve some of the problems faced by pharmaceutical manufacturers (e.g. incompatibilities), as well as offer marked benefits for patients in need of chronic treatment.

For such preservative-free formulations suitable multi-dose dispensers has to be used that will be able to prevent microbial contamination. Several technical solutions are available nowadays based on different mechanisms mainly dealing with the inhibition of contamination either via the orifice or the venting air.

Rexam's Novelia^{™} or Opthalmic pump system 750, Spectrum's Thea pharmaceuticals ABAC, Aptar'sPharmaOpthalmic Squeeze dispenser, Ursapharm'sComod-system, Adelphi Healthecare Packaging Aero Pump's 3K system, Mystic Pharmaceuticals VersiDoser and VRx2 and Pfizer's Airless Antibacterial Dispensing System are some of the already marketed devices.

The need therefore exists into developing an efficient ophthalmic multi-dose formulation lacking an antimicrobial preservative compound as BAC in a suitable container which retains sufficient sterile conditions for a suitable time period.

Aqueous ophthalmic solutions are manufactured by adding all ingredients into a solution and sterilization of said solution. Methods of sterilization known in the art, such as dry heat, steam under pressure and gas sterilization can be used to make the pharmaceutical compositions for ophthalmic use sterile. According to the present invention, sterility of prostaglandin composition is best achieved through aseptic filtration using a sterile membrane filter and filtering into a sterile container. Sterile filtration, however, has been proven the most effective method of sterilization of ophthalmic solutions containing prostaglandin.

Sterilisation by filtration is the most secure and suitable method used in pharmaceutical solutions, which do not withstand high temperatures. This method relies on the use of small diameter filters (usually <a 0.22), which hold larger microorganisms by preventing entry into the solution. Alongside containers and individual sections of the final package sterilized by an appropriate method such as oven with steam or hot air (depending on the material) and the filling is done directly after filtration of the solution and the entire process is carried out under aseptic conditions in sterile area.

According to the present invention multi-dose pharmaceutical compositions with an adequate formulation lacking antimicrobial preservative compound as Benzalkonium chloride in a suitable container which retains sufficient sterile conditions for a suitable time period, require a tuned drop size, osmolality, pH value, specific gravity and viscosity in order to exhibit similar corneal epithelial permeability as BAC contained solutions.
The present invention relates to the range of the drop size, osmolality, pH value, specific gravity and viscosity that can be achieved using formulations and various containers with preservation capabilities, which may be required for a preservative-free ophthalmic solution in order to present a similar corneal epithelial permeability compared to a BAC contained ophthalmic solution with the same active pharmaceutical ingredient.

According to the present invention, the weight ratio of the active ingredient is from about 0.0045% to about 2% by weight. In addition, a pH adjusting agent, such as hydrochloric acid or sodium hydroxide is used.

The following examples illustrate preferred embodiments in accordance with the present invention without limiting the scope or spirit of the invention:

### EXAMPLES

### Example 1: Formulation 1 of Bimatoprost 0.3 mg/ml ophthalmic solution

| **Ingredients** | **Function** | **Composition (mg/mL)** | **Ratio (% w**/**v)** |
|---|---|---|---|
| Bimatoprost | Active substance | 0.3 | 0.03 |
| Citric acid monohydrate | Buffering agent | 0.14 | 0.014 |
| Disodium hydrogen Phosphate heptahydrate | Buffering Agent | 2.68 | 0.27 |
| Sodium Chloride | Isotonic Agent | 8.3 | 8.3 |
| NaOH or HCl 1N | pH adjuster | Up to pH=7.3 | Up to pH=7.3 |
| Water for Injection | Solvent | 1 ml | qs to 100% |

The above formulation is prepared according to the following manufacturing process:
Citric acid monohydrate, disodium hydrogen phosphate heptahydrate and sodium chloride are dissolved in 80% of the final volume of water for injection at about 25-30°C. The solution is stirred for 15 min. Bimatoprost is added and stirring continues for about 30 min at 25-30°C. The residual amount of water for injection is added and the solution is stirred for an additional 5 min at about 25-30°C.
Aseptic filtration of the final solution follows through PES 0.2 µm membrane filter.

The filtrate is filled into 10 ml plastic containers such as polypropylene (PP) and polyethylene (PE) containers with Novelia^{™} of Rexam closing tip system suitable for preservative-free compositions.

Assay of the final preparations verified the complete solubilization of bimatoprost.

The solution was clear in all phases of the experiment before as well as after filtration.

Several physiochemical characteristics, including pH. osmolality, drop size and viscosity were measured for the abovementioned formulation and were compared to the marketed product's Lumigan^{®} containing bimatoprost, benzalkonium chloride, sodium chloride, citric acid monohydrate, sodium phosphate dibasic heptahydrate and purified water.
The results are shown in the below Table 1a and 1b.

**Table 1a: Physicochemical characteristics of Bimatoprost BAC-free formulation according to the present invention and Lumigan^{®}.**

| | **pH** | **osmolality (mOsm/kg)** | **Viscosity (cP)** |
|---|---|---|---|
| **Lumigan^{®}** | 7.3 | 290 | 1.29-1.33 |
| **Bimatoprost BAC-free** | 7.3 | 292 | 1.28-1.31 |

**Table 1b: Drop size of Bimatoprost BAC-free formulation according to the present invention and Lumigan^{®}.**

| | **Lumigan^{®}** | | **Bimatoprost BAC-free** | |
|---|---|---|---|---|
| | **(mg)** | | **(mg)** | |
| Min. | 28,22 | 27,25 | 30,85 | 24,5 |
| Max. | 35,38 | 36,25 | 32,9 | 32,28 |
| Mean | **33,03** | **33,99** | **32,06** | **30,34** |

| | | | | |
|---|---|---|---|---|
| Table 1b encloses the results of 2x20 drop measurements conducted by two different analysts. | | | | |

### Example 2: Formulation 2 of Latanoprost 0.005% w/v ophthalmic solution.

| **Ingredients** | **Function** |
|---|---|
| Latanoprost | Active substance |
| Sodium Chloride | Isotonic Agent |
| Disodium Phosphate anhydrous | Buffering Agent |
| Sodium Dihydrogen Phosphate monohydrate | Buffering Agent |
| NaOH or HCl 1N | pH adjuster |
| Water for Injection | Solvent |

The above formulation is prepared according to the following manufacturing process: In 85% of the water for injection sodium chloride, disodium phosphate anhydrous and sodium dihydrogen phosphate monohydrate are dissolved one by one under agitation. After complete dissolution of the above ingredients, latanoprost is added and dissolved under agitation. The pH of the resulting solution is checked and is adjusted with HCl or NaOH solutions when necessary. As a final step, the resulting solution is adjusted to the desire volume in accordance with the formula.
Assay of the final preparations verified to complete solubilization of Latanoprost.
Aseptic filtration of the final solution follows through filter membrane.

The product is filled into plastic containers such as polypropylene (PP) and polyethylene (PE) containers with Novelia^{™} of Rexam closing tip system suitable for preservative-free compositions.

### Example 3: Formulation 3 of Dorzolamide ophthalmic solution.

| **Ingredients** | **Function** |
|---|---|
| Dorzolamide HCl | Active substance |
| Mannitol | Tonicity agent |
| Sodium Citrate | Isotonic Agent |
| Hydroxyethyl cellulose | Viscosity enhancer |
| NaOH or HCl 1N | pH adjuster |
| Water for Injection | Solvent |

Ophthalmic solutions of composition 3 of Example 3 were prepared according to the following manufacturing process:
In water for injection, hydroxyethyl cellulose is dissolved in a first tank and in a second tank dorzolamide HCL, mannitol and sodium citrate are dissolved one by one under agitation. The pH of the mixture in the second tank is checked and is adjusted with HCl or NaOH solutions when necessary After complete dissolution of the above ingredients, the first mixture is added in the second mixture and dissolved under agitation. As a final step, the resulting solution is adjusted to the desire volume in accordance with the formula. Assay of the final preparations verified to complete solubilization of Dorzolamide. Aseptic filtration of the final solution follows through filter membrane.
The product is filled into plastic containers suitable for preservative-free compositions.

### Example 4: Formulation 4 of Dorzolamide + Timolol maleate ophthalmic solution.

| **Ingredients** | **Function** |
|---|---|
| Dorzolamide HCl | Active substance |
| Timolol maleate | Active substance |
| Mannitol | Tonicity agent |
| Sodium Citrate | Isotonic Agent |
| Hydroxyethyl cellulose | Viscosity enhancer |
| NaOH or HCl 1N | pH adjuster |
| Water for Injection | Solvent |

Ophthalmic solutions of composition 4 of Example 4 were prepared according to the manufacturing process used in Example 3.

### Example 5: Formulation 5 of sodium cromoglicate 20mg/ml ophthalmic solution.

| **Ingredients** | **Function** | **Composition (mg/ml)** | **Ratio (% w/v)** |
|---|---|---|---|
| Sodium Cromoglicate | Active substance | 20 | 2 |
| Disodium edetate | Chelating agent | 0.1 | 0.01 |
| Water for Injection | Solvent | qs to 1 ml | qs to 100% |

Disodium edetate is dissolved in 8 L water for injection at 30-35°C. Sodium cromoglicate is added and dissolved in a period of about 10 minutes. pH adjustment is performed if needed and the final solution and volume is made up to 10L.

Aseptic filtration of the final solution follows through 0.22µm PVDF hydrophilic filter.

The filtrate is filled into 10 ml plastic containers such as polypropylene (PP) and polyethylene (PE) containers with Novelia^{™} of Rexam closing tip system suitable for preservative-free compositions.

The solution was clear in all phases of the experiment before as well as after filtration.

As in example 1, the below physiochemical characteristics were measured for the abovementioned formulation and were compared to the marketed product's Opticrom^{®} Hayfever containing sodium cromoglicate, disodium edetate and purified water.
The results are shown in the below Table 2a and 2b.

**Table 2a: Physicochemical characteristics of Sodium cromoglicte BAC-free formulation according to the present invention and Opticrom^{®} Hayfever.**

| | **pH** | **Osmolality (mOsm/kg)** | **Viscosity (cP)** |
|---|---|---|---|
| **Opticrom^{®}Hayfever^{®}** | 5.1 | 72 | 1.98-2.00 |
| **Sodium cromoglicate BAC-free** | 5.1 | 69 | 1.35 |

**Table 2b: Drop size of Sodium cromoglicate BAC-free formulation according to the present invention and Opticrom^{®} Hayfever.**

| | **Opticrom^{®} Hayfever** | | **Sodium Cromoglicate BAC-free** | |
|---|---|---|---|---|
| | **(mg)** | | **(mg)** | |
| Min. | 31,20 | 31,20 | 35,12 | 34,97 |
| Max. | 37,10 | 36,90 | 40,61 | 38,98 |
| Mean | **34,97** | **34,81** | **37,79** | **37,83** |

Moreover, sodium cromoglicate BAC-free formulation according to the present invention was studied for its stability in normal (***25°C*/*60% RH***) and accelerated (***40°C*/*75% RH***) environmental conditions and exhibited satisfactory results raising no concerns.
In Table 2c and 2d, it is shown a summary of the stability studies conducted with regards to the physicochemical characteristics of concern.

**Table 2c: Stability studies results in normal (25°C/60% RH) environmental conditions of sodium cromoglicate BAC-free formulation according to the present invention.**

| **Test** | **Specifications** | **1 month** | **2 months** | **3 months** | **6 months** | **12 months** |
|---|---|---|---|---|---|---|
| **pH** | 4.5-6.5 | 4.8 | 4.9 | 4.9 | 4.8 | 4.5 |
| **Osmolality (mOsm/kg)** | 60-80 | 68 | 72 | 72 | 71 | 71 |
| **Drop size (S/M/E) (µL)** | *35* ± *10%* | N/A | N/A | N/A | N/A | 36.39/35.95/35.88 |
| | | | | | | ***36.07*** |
| **Viscosity (cP)** | 1.98-2.00 | N/A | N/A | N/A | N/A | 1.35 |

**Table 2d: Stability studies results in accelerated (40°C/75% RH) environmental conditions of sodium cromoglicate BAC-free formulation according to the present invention.**

| **Test** | **Specifications** | **1 month** | **2 months** | **3 months** | **6 months** |
|---|---|---|---|---|---|
| **pH** | 4.5-6.5 | 4.7 | 4.7 | 4.8 | 4.8 |
| **Osmolality (mOsm/kg)** | 60-80 | 69 | 72 | 72 | 72 |
| **Drop size (S/M/E) (µL)** | *35* ± *10%* | N/A | N/A | N/A | 38.44/38.68/37.08 |
| | | | | | ***38.07*** |
| **Viscosity (cP)** | 1.98-2.00 | N/A | N/A | N/A | N/A |

### Example 6: Formulation 6 of Brimonidine 2.0 mg/ml ophthalmic solution.

| **Ingredients** | **Function** | **Composition (mg/mL)** | **Ratio (% w/v)** |
|---|---|---|---|
| Brimonidine | Active substance | 2.0 | 0.20 |
| Polyvinyl alcohol | Viscosity-increasing agent. | 43.0 | 4.30 |
| Sodium chloride | Tonicity agent | 6.2 | 0.62 |
| Sodium citrate dihydrate | Isotonic Agent | 4.5 | 0.45 |
| Citric acid monohydrate | Buffering Agent | 0.50 | 0.05 |
| NaOH or HCl 1N | pH adjuster | pH=5.6-6.6 | pH=5.6-6.6 |
| Water for Injection | Solvent | 1 ml | qs to 100% |

The above formulation is prepared according to the following manufacturing process:
90 ml water for injection is placed in a clear vessel and stirred at about 320rpm, at 25-30°C. Brimonidine tartrate is added and the mixture is stirred for about 10 minutes at 25-30°C. Polyvinyl alcohol is added and the mixture is stirred for about 60 minutes at 25-30°C. Subsequently, sodium citrate dihydrate and citric acid monohydrate are added and stirred until a clear solution is obtained (about 10 min). Finally, sodium chloride is added and the mixture is stirred until a clear solution is obtained (about 10 min). pH adjustment is performed with aq. NaOH 1N to pH=6.2. The final volume is adjusted by adding water for injection.
Aseptic filtration of the final solution follows through PES 0.2 µm membrane filter.

The filtrate is filled into 10 ml plastic containers such as polypropylene (PP) and polyethylene (PE) containers with Novelia^{™} of Rexam closing tip system suitable for preservative-free compositions.

Assay of the final preparations verified the complete solubilization of brimonidine tartrate.

The solution was clear in all phases of the experiment before as well as after filtration.

As in example 1, the below physiochemical characteristics were measured for the abovementioned formulation and were compared to the marketed product's Alphagan^{®} containing brimonidine, benzalkonium chloride, poly(vinyl alcohol), sodium chloride, sodium citrate, citric acid monohydrate, purified water, hydrochloric acid or sodium hydroxide.
The results are shown in the Table 3a and 3b.

**Table 3a: Physicochemical characteristics of Brimonidne BAC-free formulation according to the present invention and Alphagan^{®}.**

| | **pH** | **Osmolality (mOsm/kg)** | **Viscosity (cP)** |
|---|---|---|---|
| **Alphagan^{®}** | 6.2 | 305 | 3.57 |
| **Brimonidine tartrate BAC-free** | 6.2 | 298 | 3.41 |

**Table 3b: Drop size of Brimonidine BAC-free formulation according to the present invention and Alphagan^{®}.**

| | **Alphagan^{®} (mg)** | | **Brimonidine tartrate BAC-free (mg)** | |
|---|---|---|---|---|
| Min. | 25,76 | 28,30 | 23,9 | 23,6 |
| Max. | 33,00 | 31,95 | 28,12 | 26,41 |
| Mean | **30,63** | **30,79** | **25,87** | **25,13** |

All compositions were tested for their pH value, viscosity, specific gravity and osmolality and all values were satisfying.
While the present invention has been described with respect to the particular embodiments, it will be apparent to those skilled in the art that various changes and modifications may be made in the invention without departing from the spirit and scope thereof, as defined in the appended claims.

## Claims

1. An ophthalmic pharmaceutical composition for topical administration comprising a therapeutically effective quantity of an active ingredient and/or combination with another active ingredient for the treatment of a condition affecting the exterior ocular surface, wherein said active ingredient is selected from a prostaglandin or a pharmaceutically acceptable salt or derivative thereof, a fluoroquinolone such as Levofloxacin and salts or derivatives thereof, a carbonic anhydrase inhibitor, such as Dorzolamide or salts thereof, a beta-adrenergic receptor antagonist, such as Timolol or salts thereof, a mast cell stabilizer such as cromoglicic acid or salts thereof, an α₂-adrenoceptor antagonist such as brimonidine or salts thereof and/or combination of said active ingredients, and further comprising an effective amount of agents for the optimization of the drop size, the pH value, the osmolality and viscosity in order to improve the stability and permeability properties of the composition, and without the use of any antimicrobial preservative compound such as benzalkonium chloride.

2. The ophthalmic pharmaceutical composition according to claim 1, wherein said prostaglandin is selected from the group of bimatoprost, latanoprost, and travoprost.

3. The ophthalmic pharmaceutical composition according to any preceding claim, wherein it further comprises a pH adjusting agent, such as hydrochloric acid or sodium hydroxide.

4. The ophthalmic pharmaceutical composition according to any preceding claim, wherein it further comprises a buffering agent, such as disodium hydrogen phosphate heptahydrate and citric acid monohydrate, and an isotonic agent, such as sodium chloride or sodium citrate.

5. A process for the preparation of an ophthalmic pharmaceutical composition for topical administration comprising a therapeutically effective quantity of an active ingredient and/or combination with another active ingredient for the treatment of a condition affecting the exterior ocular surface, wherein said active ingredient is selected from a prostaglandin or a pharmaceutically acceptable salt or derivative thereof, a fluoroquinolone such as Levofloxacin and salts or derivatives thereof, a carbonic anhydrase inhibitor, such as Dorzolamide or salts thereof, a beta-adrenergic receptor antagonist, such as Timolol or salts thereof, a mast cell stabilizer such as cromoglicic acid or salts thereof, an α₂-adrenoceptor antagonist such as brimonidine or salts thereof and/or combination of said active ingredients and further comprising an effective amount of agents for the optimization of the drop size, the pH value, the osmolality and viscosity in order to improve the stability and permeability properties of the composition, and without the use of any antimicrobial preservative compound such as benzalkonium chloride, said composition is being sterilized by aseptic filtration through membrane filter.
